# EUROPEAN PATENT SPECIFICATION

(11) **EP 0 964 058 B1**
(45) Date of publication and mention of the grant of the patent: **31.05.2006**
(21) Application number: 99201289.8
(22) Date of filing: 27.04.1999
(51) Int. Cl.: C12P 7/64

(54) **Process for the enrichment of compounds in trans-10 isomers**
Verfahren zur Anreicherung von Trans-10 Isomeren
Procédé d'enrichissement de trans-10 isomères

(30) Priority: 12.05.1998 EP 98201580
(43) Date of publication of application: 15.12.1999
(73) Proprietor: LODERS CROKLAAN B.V., 1521 AZ Wormerveer (NL)
(72) Inventor: Cain, Frederick William, 1521 AZ Wormerveer (NL); van Hoek, Gerlof Louwrens Maarten, 7441 KC Nijverdal (NL); Taran, Victoria, 1521 AZ Wormerveer (NL)
(74) Representative: Stevens, Ian Edward

(56) References cited:
- WO-A-96/37586
- WO-A-96/37587
- WO-A-97/18320
- CHEMICAL ABSTRACTS, vol. 112, no. 19, 7 May 1990 (1990-05-07) Columbus, Ohio, US; abstract no. 174637, HILLS, MATTHEW J. ET AL: "Lipase from Brassica napus L. discriminates against cis-4 and cis-6 unsaturated fatty acids and secondary and tertiary alcohols" XP002081632 & BIOCHIM. BIOPHYS. ACTA (1990), 1042(2), 237-40 CODEN: BBACAQ;ISSN: 0006-3002,
- CHEMICAL ABSTRACTS, vol. 111, no. 9, 28 August 1989 (1989-08-28) Columbus, Ohio, US; abstract no. 73700, ZOTTOR, B. A. K. ET AL: "Relative hydrolysis of trans- and cis-triglyceride by rat mammary lipoprotein lipase" XP002081633 & NUTR. RES. (N. Y.) (1989), 9(6), 679-83 CODEN: NTRSDC;ISSN: 0271-5317,

## Description

In our earlier patent application WO 97/18320 we have disclosed a method, based on alcoholysis (so including glycerolysis) for the enrichment of a compound (such as CLA) comprising different geometrical isomers of polyunsaturated fatty acids in one of these isomers by subjecting a starting composition to an enzymatic treatment with an enzyme having specificity for one of these isomers. This alcoholysis route enables us to make compositions that are enriched in specific desired isomers, including trans-10 isomers. However above process is not very suitable to obtain an ester product that is enriched in trans-10 isomers. This is caused by the fact that the enzymes disclosed in this earlier patent application can only be used for an enrichment in trans-10 isomers along the free fatty acid route. If an enrichment in the trans-10 isomer is required in the esters first a free fatty acid product has to be made that is then converted into the esters and this process has to be repeated several times. This makes that this process is complicated and leads to lower enrichment. Therefore we have studied whether we could find other routes with which an ester product, enriched in trans-10 isomers could be made in one esterification step resulting in higher enrichment.

WO 96/37587 and WO 96/37586 disclose processes for the production of materials enriched in long chain polyunsaturated fatty acids.

Chemical Abstracts (XP 002081632) discloses that the lipase from *Brassica napus L.* discriminates between cis-4 and cis-6 unsaturated fatty acids and secondary and tertiary alcohols.

Chemical Abstracts (XP 002081633) discloses discrimination between trans- and cis-triglycerides by rat mammary lipoprotein lipase.

Above study resulted in our invention, wherein known enzymes are applied from which it was not known that they have trans-10 specificity.
Therefore our invention concerns in the first instance a process according to claim 1 for the enrichment of a polyunsaturated fatty acid (=PUFA) mixture, comprising different isomers with at least two conjugated unsaturations, including isomers from which one unsaturation is a trans-10 double bond,
the PUFA-mix in particular being a CLA-mixture, comprising different CLA-isomers, but at least including CLA-isomers with a trans-10 double bond,
in polyunsaturated isomers having a trans-10 double bond, in particular in CLA-isomers with a trans-10 double bond,
wherein the PUFA-mix comprising at least 5 wt% of trans-10 isomer, in particular at least 5% of the trans-10 CLA-isomer is subjected to an enzymatic conversion with a mono-, di-or higher alcohol using an enzyme that can discriminate trans-10 isomers from other cis and/or trans isomers also present in the PUFA-mix, in particular the CLA mixture
and separating the mixture obtained after the conversion into unconverted PUFA-acids, in particular CLA and esters or glycerides from the PUFA-acids, in particular the CLA by physical or chemical means
and isolating an ester or glyceride mix from PUFA's, in particular from CLA's that is enriched in the trans-10 PUFA-isomers, in particular the CLA-isomers with at least 10 %, preferably at least 20 % more preferably at least 30 % compared to the starting mixture.

Our new process is particularly useful, when using short alkyl alcohols (C1-C6), preferably ethanol and glycerol, that are foodgrade.

Enzymes that are preferred for our purposes are
1) lipases derived from Alcaligenes and having a molecular weight measured by gel filtration of less than 250,000 or
2) a lipase derived from Pseudomonas cepacia.
These enzymes are commercially available eg from Meito Sangyo CO. However it was not known that these enzymes can discriminate trans-10 isomers from other cis and trans isomers.

The process is performed at 20-80 °C, preferably at 30-55 °C most preferably at 40-50 °C.
The water formed during this process can be removed from the reaction mix. Methods to do this are well known and include a vacuo treatment, or the use of a water binder such as a molecular sieve or the use of an inert stripping gas.
In this way the water content of the reaction mix can be maintained at 1-4 wt% during the conversion.

The separation of unconverted PUFA-isomers, in particular the CLA-isomers and PUFA-esters, in particular the CLA-esters formed is performed by molecular destillation and/or by forming salts of the free PUFA-isomers, in particular CLA-isomers and removal of the salts formed.

The starting materials applied for the conversion are preferably based on a PUFA-mix, in particular a CLA-mix containing more than 20 wt%, preferably more than 30 wt % of the trans-10 PUFA-isomers, in particular the trans-10 CLA-isomers, based on the total PUFA-mix, in particular the total CLA-mix.

Particularly preferred starting materials comprise trans-10cis-12 isomers and cis-9trans-11 isomers in about equimolecular amounts.

The processing can be performed until a limited conversion is achieved or until at least one of the components of the reaction mix is completely converted. This will depend on the fact whether the mole ratio PUFA-isomers (or CLA isomers) : alcohol applied is equivalent or not. In case the mole ratio PUFA-isomers : alcohol is above equimolecular the conversion will be about completed. If however this ratio is below equimolecular this conversion will be limited to below 80 %, preferably below 70 %, of PUFA-isomers applied.

### EXAMPLES

### 1. Preparation of starting composition (CLA 1:1 mix of c9t11 and t10c12).

150 G of NaOH was dissolved in 1600 g of propyleneglycol by heating at 60 °C. Than the temperature was increased to 90°C and 523 g of safflower oil was added to this mix. After that the temperature was raised until 135 °C. The mixture obtained was stirred at 135°C under N2 for 40 hrs. The mixture was cooled to 90°C and the soap formed was split with 1.2 1 sulphuric acid (10 Vol %). The pH of the mixture was 3.0. The oil obtained was washed three times with hot water, each time with 1 1 of water. pH of the water after last washing was 7.0. The oil was dried under vacuum at 80°C. 420 G of CLA was obtained. There was no water detected by Karl Fisher method in the end product. The composition of this CLA (measured by high resolution FAME) was:

| CARBON CHAIN | % |
|---|---|
| C16:0 | 6.77 |
| C18:0 | 2.70 |
| C18:1 | 14.34 |
| C18:2 | 6.01 |
| c9t11 | 33.81 |
| CLA1012CT | 0.39 |
| t10c12 | 33.66 |
| others CLA isomers | 2.08 |
| C22:0 | 0.24 |

### II. Enrichment in t10c12 isomer using different enzymes

100 G of the CLA obtained in example I was mixed with 0.5 g of water,
Samples of 10 g of above CLA/water mix were mixed with different lipases (0.5 wt % of enzyme on CLA) and the reaction was started by adding 2.02 ml of ethanol (96 %) to each sample. The reaction mixture was stirred at 38°C and after 2;4 and 24 hrs samples were taken and analysed. Herefore 1 ml of each sample was mixed with 1 ml of water and 2 ml of iso-octane. The mixture was strongly stirred for 1 min and the extracted oil in iso-octane was collected.
After removal of the iso-octane the samples were analysed on free fatty acid content by titration with 0.2 N NaOH and on isomer content after conversion of the ethyl esters into methyl esters by FAME analysis.
The results are summarised in the table below:

**TABLE Lipase applied conversion conv.time ffa c9t11 t10c12 c9t11/t10c12**

| | % | hours | % | % | | |
|---|---|---|---|---|---|---|
| ALCALIGENES spp* | 14.6 | 2 | 84.9 | 20.3 | 50.5 | 29:71 |
| | 30.8 | 4 | 68.8 | 23.0 | 48.0 | 32:68 |
| | 49.4 | 24 | 50.3 | 26.5 | 42.9 | 38:62 |
| SPEUDOMONES* | 24.4 | 2 | 81.5 | 26.4 | 41.7 | 39:61 |
| | 35.2 | 4 | 69.8 | 27.2 | 41.1 | 40:60 |
| | 58.8 | 24 | 44.4 | 29.8 | 38.2 | 44:56 |

| | | | | | | |
|---|---|---|---|---|---|---|
| * enzymes from Meito Sankyo Codes resp QL and SL | | | | | | |

## Claims

1. Process for the enrichment of a polyunsaturated fatty acid (=PUFA) mixture, comprising different isomers with at least two conjugated unsaturations, including isomers from which one unsaturation is a trans-10 double bond,
wherein the PUFA-mix comprising at least 5 wt% of trans-10 isomer is subjected to an enzymatic conversion with a mono-di-or higher alcohol using an enzyme that can discriminate trans-10 isomers from other cis and/or trans isomers also present in the PUFA-mix
wherein the enzyme is a lipase derived from Alcaligenes and having a molecular weight measured by gel filtration of less than 250,000 or is a lipase derived from Pseudomonas cepacia
and separating the mixture obtained after the conversion into unconverted PUFA-acids and esters or glycerides of the PUFA-acids by physical or chemical means
and isolating an ester or glyceride mix that is enriched in the trans-10 PUFA-isomers with at least 10 %, preferably at least 20 % more preferably at least 30% compared to the starting mixture.

2. Process according to Claim 1, wherein the PUFA-mix is a conjugated linoleic acid (CLA) mixture comprising different CLA - isomers including at least 5 Wt % of a trans-ID CLA - isomer.

3. Process according to claims 1-2, wherein the conversion is performed at 20-80 °C, preferably at 30-55 °C most preferably at 40-50 °C.

4. Process according to claims 1-3, wherein water formed during the conversion is removed, preferably by vacuo or by adding a water binder, in particular a molecular sieve.

5. Process according to claims 1-4 wherein the water content of the reaction mixture is maintained at 1-4 wt% during the conversion.

6. Process according to claims 1-5, wherein the separation of unconverted PUFA-isomers and PUFA-esters formed is performed by molecular destillation and/or by forming salts of the free PUFA-isomers and removal of the salts formed.

7. Process according to Claim 6, wherein the PUFA-isomers are CLA-isomers and the PUFA-esters are CLA-esters.

8. Process according to claims 1 to 7, wherein the PUFA-mix used as starting material contains more than 20 wt %, preferably more than 30 wt % of the trans-10 PUFA-isomers based on the total PUFA-mix.

9. Process according to Claim 8, wherein the PUFA-mix is a CLA mixture and the trans-10 PUFA-isomers are trans-10 CLA-isomers.

10. Process according to claims 1 to 9, wherein the CLA mixture used as starting material comprises trans-10 cis 12 and cis-9trans-11 isomers in about equimolecular amounts.

11. Process according to claims 1 to 10, wherein a mole ratio PUFA-isomers: (mono, -di-or polyalcohol) above the equimolecular ratio is applied while the PUFA-conversion is about completed.

12. Process according to claim 11, wherein the PUFA-isomers are CLA-isomers and the PUFA-conversion is a CLA-conversion.

13. Process according to claims 1 to 12, wherein a mole ratio PUFA-isomers (in particular CLA-isomers) : (mono-,di-or polyalcohol) below the equimolecular ratio is applied, while the conversion of the PUFA-isomers CLA-isomers is kept below 80 %, preferably below 70 %.

## Patentansprüche

1. Prozess zur Anreicherung einer mehrfach ungesättigten Fettsäuren(= MUFS)-Mischung, die unterschiedliche Isomere mit mindestens zwei konjugierten Unsättigungen aufweist, einschließlich isomeren, bei denen eine Unsättigung eine trans-10-Doppelbingung ist,
wobei die MUFS-Mischung, die mindestens 5 Gewichts-% an trans-10-Isomer aufweist, einer enzymatischen Umwandung mit einem mono-, di- oder höherem Alkohol unter Verwendung eines Enzyms unterworfen wird, das trans-10-Isomere von anderen cis- und/oder trans-Isomeren, unterscheiden kann, die ebenfalls in der MUFS-Mischung vorliegen,
wobei das Enzym eine Lipase ist, die von Alkaligenen erhalten wird und ein Molekulargewicht gemessen durch Gelfiltration von mindestens 250.000 aufweist, oder eine Lipase ist, die von Pseudomonas cepacia erhalten wird,
und wobei die Mischung, die nach der Umwandlung erhalten wird, in nicht umgewandelte MUFS-Säuren und Ester oder Glyceride der MUFS-Säuren durch physikalisch oder chemische Mittel aufgetrennt wird,
und wobei eine Ester- oder Glyceridmischung, die verglichen mit der Ausgangsmischung in Bezug auf die trans-1D-MUFS-Isomere mit mindestens 10 %, vorzugsweise mindestens 20 %, mehr bevorzugt mindestens 30 % angereichert ist, isoliert wird.

2. Prozess nach Anspruch 1, wobei die ML1FS-Mischung eine konjugierte Linolsäure(=KLS)-Mischung ist, die unterschiedliche KLS-Isomere einschließlich mindestens 5 Gewichts-% eines trans-10-KLS-Isomers aufweist.

3. Prozess nach Anspruch 1 bis 2, wobei die Umwandlung bei 20 bis 80 °C, vorzugsweise bei 30 bis 55 °C, am meisten bevorzugt bei 40 bis 50 °C, durchgeführt wird.

4. Prozess nach Anspruch 3, wobei Wasser, das während der Umwandlung gebildet wird, entfernt wird, vorzugsweise durch Vakuum oder durch Zugabe eines Wasserbinders, insbesondere eines Molekularsiebs.

5. Prozess nach Anspruch 1 bis 4, wobei der Wassergehalt der Reaktionsmischung während der Umwandlung bei 1 bis 4 Gewichts-% gehalten wird.

6. Prozess nach Anspruch 1 bis 5, wobei die Auftrennung von nicht umgewandelten MUFS-Isomeren und MUFS-Estern, die gebildet wurden, durch Molekulardestillation und/oder durch Bildung von Salzen von freien MUFS-Isomeren und Entfernung der gebildeten Salze durchgeführt wird.

7. Prozess nach Anspruch 6, wobei die MUFS-Isomere KLS-Isomere und die MUFS-Ester KLS-Ester sind.

8. Prozess nach Anspruch 1 bis 7, wobei die MUFS-Mischung, die als Ausgangsmaterial verwendet wird, basierend auf der gesamten MUFS-Mischung mehr als 20 Gewichts-%, vorzugsweise mehr als 30 Gewichts-%, der trans-10-MUFS-Isomere enthält.

9. Prozess nach Anspruch 8, wobei die MUFS-Mischung eine KLS-Mischung ist und die trans-10-MUFS-Isomere trans-10-KLS-Isomere sind.

10. Prozess nach Anspruch 1 bis 9, wobei die KLS-Mischung, die als Ausgangsmaterial verwendet wird, trans-10cis12- und cis-9trans-11-Isomere in ungefähr equimolaren Mengen aufweist.

11. Prozess nach Anspruch 1 bis 10, wobei ein Molverhältnis MUFS-Isomere:(Mono-, Di- oder Polyalkohol) oberhalb des equimolekularen Verhältnisses angewandt wird, während die MUFS-Umwandlung abgeschlossen wird.

12. Prozess nach Anspruch 11, wobei die MUFS-Isomere KLS-Isomere sind und die MUFS-Umwandlung eine KLS-Umwandlung ist.

13. Prozess nach Anspruch 1 bis 12, wobei ein Molverhältnis MUFS-Isomere (insbesondere KLS-isomere): (Mono-, Di- oder Polyalkohol) unterhalb des equimolekularen Verhältnisses angewandt wird, während die Umwandlung der MUFS-Isomere bzw. KLS-Isomere unter 80 %, vorzugsweise unter 70 % gehalten wird.

## Revendications

1. Procédé d'enrichissement d'un mélange d'acide gras polyinsaturé (=PUFA), comprenant différents isomères ayant au moins deux insaturations conjuguées, incluant les isomères à partir desquels une insaturation est une double liaison trans-10,
dans lequel le mélange de PUFA comprenant au moins 5 % en poids d'isomère trans-10 est soumis à une conversion enzymatique avec un mono-, un di-alcool ou un alcool supérieur en utilisant un enzyme qui peut distinguer les isomères trans-10 des autres isomères cis et/ou trans également présents dans le mélange de PUFA, dans lequel l'enzyme est une lipase dérivée des *Alcaligenes* et ayant un poids moléculaire mesuré par filtration sur gel de moins de 250 000 ou est une lipase dérivée de *Pseudomonas cepacia*,
et à une séparation du mélange obtenu après la conversion en des acides PUFA non convertis et des esters ou des glycérides des acides PUFA par des moyens physiques ou chimiques,
et à un isolement d'un mélange d'ester ou de glycéride qui est enrichi en isomères trans-10-PUFA avec au moins 10 %, de préférence au moins 20 % et plus préférablement au moins 30 % par rapport au mélange initial.

2. Procédé selon la revendication 1, dans lequel le mélange de PUFA est un mélange d'acide linoléique conjugué (CLA) comprenant différents isomères de CLA incluant au moins 5 % en poids d'un isomère trans-10-CLA.

3. Procédé selon les revendications 1-2, dans lequel la conversion est réalisée à 20-80-°C, de préférence à 30-55 °C de manière préférée entre toutes à 40-50 °C.

4. Procédé selon les revendications 1 - 3, dans lequel l'eau formée pendant la conversion est éliminée, de préférence sous vide ou en ajoutant un liant aqueux, en particulier un tamis moléculaire.

5. Procédé selon les revendications 1 - 4, dans lequel la teneur en eau du mélange réactionnel est maintenue à 1 - 4 % en poids pendant la conversion.

6. Procédé selon les revendications 1 - 5, dans lequel la séparation des isomères de PUFA non convertis et des esters de PUFA formés est réalisée par distillation moléculaire et/ou par formation de sels d'isomères de PUFA libres, et élimination des sels formés.

7. Procédé selon la revendication 6, dans lequel les isomères de PUFA sont des isomères de CLA et les esters de PUFA sont des esters de CLA.

8. Procédé selon les revendications 1 à 7, dans lequel le mélange de PUFA utilisé comme matériau de départ contient plus de 20 % en poids, de préférence plus de 30 % en poids des isomères trans-10-PUFA par rapport au mélange total de PUFA.

9. Procédé selon la revendication 8, dans lequel le mélange de PUFA est un mélange de CLA et les isomères trans-10-PUFA sont des isomères trans-10-CLA.

10. Procédé selon les revendications 1 à 9, dans lequel le mélange de CLA utilisé comme matériau de départ comprend les isomères trans-10-cis-12 et cis-9-trans-11 dans des quantités environ équimolaires.

11. Procédé selon les revendications 1 à 10, dans lequel un rapport molaire des isomères de PUFA (mono-, di- ou polyalcool) au-dessus du rapport équimolaire est appliqué tandis que la conversion de PUFA est quasiment achevée.

12. Procédé selon la revendication 11, dans lequel les isomères de PUFA sont des isomères de CLA et la conversion de PUFA est une conversion de CLA.

13. Procédé selon les revendications 1 à 12, dans lequel un rapport molaire des isomères de PUFA (en particulier des isomères de CLA) : (mono-, di- ou polyalcool) au-dessous du rapport équimolaire est appliqué, tandis que la conversion des isomères de PUFA isomères de CLA est maintenue au-dessous de 80 %, de préférence au-dessous de 70 %.
